# EUROPEAN PATENT APPLICATION

(11) **EP 4 721 856 A1**
(43) Date of publication of application: **08.04.2026**
(21) Application number: 24383084.1
(22) Date of filing: 07.10.2024
(51) Int. Cl.: B01J 23/83, B01J 37/03, B01J 37/18, C07C 31/00

(54) **NEW CATALYST FOR THE PRODUCTION OF DIOLS FROM OXYGEN-CONTAINING FIVE AND SIX MEMBERED HETEROCYCLIC COMPOUNDS**

(71) Applicant: Universidad del Pais Vasco - Euskal Herriko Unibertsitatea (UPV/EHU), 48940 Leioa (ES)
(72) Inventor: BARREDO VINUESA, Asier, 48940 Leioa (ES); VIAR ANTUÑANO, Nerea, 48940 Leioa (ES); GANDARIAS GOIKOETXEA, Iñaki, 48940 Leioa (ES); REQUIES MARTÍNEZ, Jesús María, 48940 Leioa (ES)
(74) Representative: Balder IP Law, S.L.

(57) **Abstract**

New, trifunctional catalyst, comprising three types of metals or derivatives thereof, and a process for obtaining such catalyst. The catalyst of the invention is useful for a cost-effective method comprising the ring-opening of oxygen-containing five and six membered heterocyclic compounds, to obtain diols, with a high selectivity and yield.

## Description

### TECHNICAL FIELD

The present invention relates to a new, trifunctional catalyst, comprising three types of metals or derivatives thereof, and a process for obtaining such catalyst. The catalyst of the invention is useful for a cost-effective method comprising the ring-opening of oxygen-containing five and six membered heterocyclic compounds, to obtain diols, with a high selectivity and yield.

### STATE OF THE ART

Diols are organic compounds containing two hydroxyl groups and play a crucial role in organic synthesis, particularly in the preparation of products like polyesters, polyurethanes, coatings, inks, perfumes, and plasticizers.

As an example, 1,4-butanediol functions as a solvent and is employed in the manufacture of some types of plastics, such as polybutylene terephthalate (PBT), elastic fibers and polyurethanes, as well as for the synthesis of gamma-butyrolactone.

A further example is 1,6-hexanediol, which is widely used for the production of polyester and polyurethane. The fairly long hydrocarbon chain can improve the hardness and flexibility of polyesters containing it. Therefore, it is commonly employed as a chain extender, the resulting modified polyurethane having high resistance to hydrolysis as well as mechanical strength, but with a low glass transition temperature. 1,6-hexanediol is also an intermediate to acrylics as a crosslinking agent.

The industrially most important diol may be considered 1,5-pentanediol (1,5-PDO), which is extensively used in the synthesis of diverse products such as polyesters, polyurethanes, coatings, inks, perfumes and plasticizers [J. Lu et al, ACS Sustainable Chemistry and Engineering (2017), 5 (7), 6159-6166; J. J. Tan et al, Journal of Fuel Chemistry and Technology (2021), 49 (6), 780-790]. It is indeed considered one of the most valuable chemical compounds, with a global market valued at $47.05 million in 2022, and is expected to reach $54.70 million by 2029 [source: Market Research, «https://www.marketresearch.com/QYResearch-Group-v3531/Global-Pentanediol-Research-33511018]*.* Currently, 1,5-pentanediol is produced through the catalytic hydrogenation of glutaric acid, a process that involves the use of toxic chemicals such as potassium cyanide, and relies on petroleum-derived compounds [G. Hayes et al, Chemical Reviews (2023), 123 (5), 2609-2734]. Due to the high costs and limited availability of C5 petrochemical feedstocks, the production and applications of 1,5-pentanediol remain small scale. However, 1,5-pentanediol can also be produced from furfural (FUR), a feedstock derived from biomass [Z. Yang, J. et al, Green Chemical Engineering (2021), 2 (2), pp. 158-173*].*

Furfural is a key chemical platform derived from the hydrolysis and subsequent dehydration of xylan, which is a polysaccharide present in lignocellulosic biomass [T. Zhang et al, Bioresource Technology (2022), 354, 127126]. The abundant availability, renewable nature and low cost of lignocellulosic biomass make furfural an attractive chemical platform for the production of high added value products, such as 1,5-pentanediol. Additionally, biomass-based routes are expected to have a lower CO₂ footprint compared to petroleum-based routes [Z. Yang, J. et al, Green Chemical Engineering (2021), 2 (2), pp. 158-173]. Consequently, the conversion of lignocellulosic biomass to 1,5-pentanediol is a promising approach to develop a sustainable chemical process.

1,5-pentanediol can primarily be produced by three routes from furfuryl alcohol (FAL), which is obtained from the hydrogenation of furfural (Scheme 1):
(i) hydrogenation of furfuryl alcohol to tetrahydrofurfuryl alcohol (THFA) and subsequent ring-opening hydrogenolysis [WO2018223034A1*,* CN102942448A7];
(ii) direct ring opening hydrogenolysis of furfuryl alcohol [R. G. Kurniawan et al., Applied Catalysis B: Environmental (2023), 320, 121971*];* and
(iii) sequential dehydration-hydration-hydrogenation pathway using tetrahydrofurfuryl alcohol, dihydropyran (DHP) and 2-hydroxytetrahydropan (2-HY-THP) [J. Peng et al, Fuel (2023), 332*;* WO2018170932A1*].*

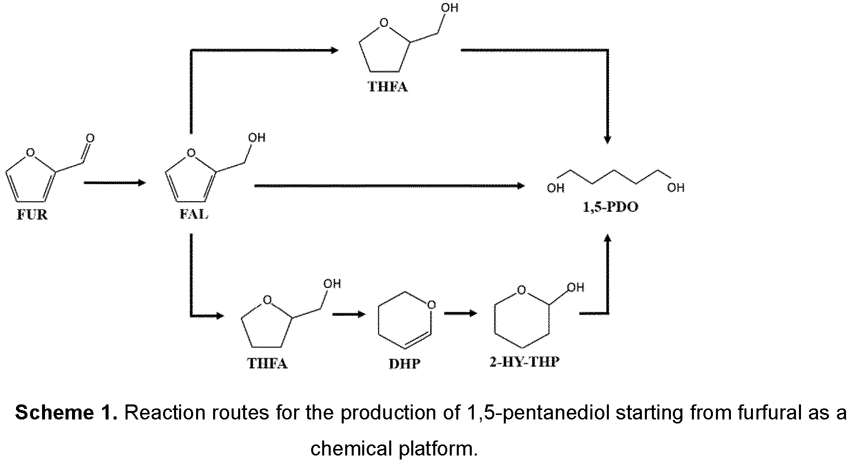

In the direct route from furfuryl alcohol to 1,5-pentanediol, it is very difficult to control the adsorption configuration of the reagent on the catalytic surface, which considerably reduces the yield of 1,5-pentanediol due to the high presence of secondary reactions. The highest performance recorded to date is achieved by Kurniawan et al. [R. G. Kurniawan et al., Applied Catalysis B: Environmental (2023), 320, 121971*],* who developed a Ni⁰/CoOₓ/Al₂O₃ catalyst that provided a 1,5-pentanediol yield of 47.5% for 160 °C, 3 MPa of H₂ and 6 hours of reaction time, using ethanol as a solvent. Conversely, the route with DHP as an intermediate involves 4 consecutive reaction steps and requires the suppression of secondary reactions, which prevents achieving high productivity of 1,5-pentanediol [J. Peng et al, Fuel (2023), 332].

In contrast to these routes, hydrogenation of furfuryl alcohol to tetrahydrofurfuryl alcohol and subsequent ring opening allows for higher selectivity synthesis of 1,5-pentanediol. Hydrogenation of furfuryl alcohol to tetrahydrofurfuryl alcohol is easily performed with simple Ni-based catalysts. For ring opening of tetrahydrofurfuryl alcohol, multiple studies have investigated the catalytic synergistic effect between the hydrogenation active site (e.g., Pt, Rh, Pd, Ir, and Ru) and the strong oxophilic site (e.g., MoOₓ, WOₓ and ReOₓ) [WO2018170932A1*;* CN111229204A*].* However, the high price of noble metals used in this type of catalyst (e.g., Rh: 155 USD g⁻¹, Ir: 174.3 USD g⁻¹, Re: 2.02 USD g⁻¹) greatly reduces the scalability of the process. Other studies have achieved ring opening of tetrahydrofurfuryl alcohol using non-noble catalysts [WO2013073705A1*;* WO2013073705A1]. However, they require more severe operating conditions (e.g. T= 250 °C, P= 250 bar), or achieve only a low selectivity to 1,5-pentanediol (≃ 50 %).

WO2018223034A1 discloses the use of a catalyst based on the noble metals Ru, Rh and Ir, obtaining a yield of around 0.25 t_{product} t_{cat}⁻¹ h⁻¹ in the conversion of tetrahydrofurfuryl alcohol to 1,5-pentanediol. However, when operating with water as a solvent, the scalability of the process is significantly reduced, since larger equipment is required to handle the same amount of reagent as in pure streams, in addition to higher energy costs for recovering the desired product from the diluted stream. Furthermore, the high price of the noble metals used (15.2 USD g⁻¹, Rh: 155 USD g⁻¹ and Ir: 174.3 USD g⁻¹) greatly increases the overall costs of the process. CN102942448A discloses the use of a Pt/WOₓ/ZrOₓ catalyst with tetrahydrofurfuryl alcohol diluted in water to 60%, obtaining productivities lower than 0.1 t_{product} t_{cat}⁻¹ h⁻¹, which, according to the study by J. Lange [Jean-Paul Lange, Catalysis Science & Technology (2016), 6, 4759], is the lower limit for industrial applicability in biorefineries. CN109896921A discloses the use of a Mo Sulfide + Pt + CeOz catalyst where no 1,5-pentanediol selectivity higher than 60% was achieved. CN111229204A, despite obtaining selectivities greater than 95% of 1,5-pentanediol, operates with 5% tetrahydrofurfuryl alcohol in water, requiring long reaction times and thus resulting in low activity values, around 0.1 t_{product} t_{cat}⁻¹ h⁻¹.

WO2013073705A1 discloses catalysts based on non-noble metals for the conversion of tetrahydrofurfuryl alcohol into 1,5-pentanediol. Although a high activity value is achieved (>0.5 t_{product} t_{cat}⁻¹ h⁻¹), the disclosed process requires more severe operating conditions of T= 260°C and P=250 bar, which greatly increases the operating cost. Conversely, KR101432638B1 discloses the use of a CuO/SiO₂ catalyst, which may also include Ni or Co as a hydrogenating metal. However, low selectivities (<70%) are obtained, due to the side reaction for the production of δ-valerolactone.

*Al-Yusufi et al. [*M. Al-Yusufi et al., ACS Sustainable Chemistry and Engineering (2022), 10 (15), 4954-4968*]* disclose a catalyst comprising nickel and rare earth metal oxide or hydroxide (Lanthanum, Praseodymium or Samarium) and *Zhao et al. [*W. Zhao et al., Fuel (2023), 354(6), 129312*]* disclose a catalyst comprising Ni and Lanthanum hydroxide; both catalysts only achieving activities as low as 0.14 and 0.04 t_{product} t_{cat}⁻¹ h⁻¹.

Despite the low cost of lignocellulosic biomass, the industrial production of 1,5-pentanediol from this biomass is significantly limited due to the difficulty of achieving an efficient conversion from furfural as the starting chemical product. Hydrogenation of furfural to tetrahydrofurfuryl alcohol and subsequent ring opening to produce 1,5-pentanediol is presented as the most promising route due to its high selectivity. However, to date, it has not been possible to obtain adequate yields using low-cost, non-noble catalysts.

Therefore, there is still a need to develop cost-effective catalysts that avoid the use of noble metals and are capable of opening oxygen-containing heterocycles, such as tetrahydrofurfuryl alcohol, with the obtained products being useful as the starting materials for many products.

### DESCRIPTION OF THE INVENTION

The present invention is directed to a trifunctional catalyst, useful for a method for ring opening of oxygen-containing heterocyclic compounds, with improved selectivity and yield, and based on non-noble metals, thus reducing the overall cost of such a method. Additionally, it allows performing the reaction of ring opening oxygen-containing heterocyclic compounds in pure form, without the need for dilution with any solvent. The possibility of performing such ring opening without the presence of a solvent, reduces the need for solvent separation from the obtained diol at the end of the reaction, thus making the process time and cost effective. Moreover, the volume of the reactor may be reduced in the absence or with limited presence of solvent. Furthermore, the ring opening may be performed at temperatures which are lower than those generally used in the art, thus further reducing the cost of the method.

Accordingly, a first aspect of the present invention relates to a catalyst comprising:
1) a first metal M1, selected from Nickel, Copper, Platinum, Ruthenium, Rhodium, Iridium, Palladium and mixtures of two or three of the foregoing;
2) a second metal M2, which is Cobalt;
3) a third metal M3, selected from Lanthanum, Cerium, Yttrium, Samarium, Praseodymium, Neodymium and mixtures of two or three of the foregoing;
wherein the first and second metals M1 and M2 may be present as metal oxides, elemental metals, or mixtures thereof, and the third metal M3 may be present as metal oxide, metal carbonate, metal hydroxide, elemental metal, or mixtures thereof.

The first metal or metals M1 have hydrogenating properties. Preferably, the first metal M1 is Nickel.

The second metal M2, which is Cobalt, has oxophilic properties.

The third metal or metals M3 have oxophilic and/or basic properties. Preferably, the third metal M3 is Lanthanum.

In the frame of the present invention, the terms "the first metal M1" or "the third metal M3" both comprise an individual metal M1 or M3, that is, either only one metal is present, or a mixture of two or three different metals, according to the above definitions.

According to particular embodiments, the first metal M1 or the sum of metals M1 is comprised in the catalyst in an amount of between 0.5 to 40 wt%, preferably between 0.5 to 30 wt%, with regard to the total weight of the catalyst. According to further particular embodiments, the first metal M1 (or sum of metals M1) is comprised in amounts of 1 wt% or more, of 2 wt% or more, of 3.5 wt% or more, of 5 wt% or more, or of 10 wt% or more; and in amounts of 25 wt% or less, of 20 wt% or less, of 15 wt% or less, or of 10 wt% or less; all the weight percentages are given with regard to the total weight of the catalyst. The listed upper and lower limits may be combined one with another.

According to particular embodiments, the second metal M2 is comprised in the catalyst in an amount of between 20 to 80 wt%, preferably between 30 and 70 wt%, with regard to the total weight of the catalyst. According to further particular embodiments, the second metal M2 is comprised in amounts of 40 wt% or more, of 50 wt% or more, or of 60 wt% or more; and in amounts of 60 wt% or less, of 50 wt% or less, or of 40 wt% or less; all the weight percentages are given with regard to the total weight of the catalyst. The listed upper and lower limits may be combined one with another.

According to particular embodiments, the third metal M3 or the sum of metals M3 is comprised in the catalyst in an amount between 10 to 80 wt%, preferably between 30 and 70 wt%, all percentages being with regard to the total weight of the catalyst. According to further particular embodiments, the third metal M3 is comprised in amounts of 40 wt% or more, of 50 wt% or more, or of 60 wt% or more; and in amounts of 60 wt% or less, of 50 wt% or less, of 40 wt% or less, or of 30 wt% or less; all the weight percentages are given with regard to the total weight of the catalyst. The listed upper and lower limits may be combined one with another.

According to a preferred embodiment, the molar ratio between the first metal M1 to the second metal M2 is preferably between 1:50 and 4:1; according to a more preferred embodiment, the molar ratio of the first metal M1 to the second metal M2 is between 1:50 to 1:1; according to even more preferred embodiments, said molar ratio is between 1:8 and 8.1, between 1:8 and 1:1, between 1:3 and 3:1, or between 1:3 and 1:1.

The oxidation states of metals M1 and M2 when present as metal oxide, may be any of the known oxidation states known for the respective metals.

According to a particular embodiment, the catalyst has the formula NiOₓ/CoO_{y}/La₂O₂CO₃, wherein x and y, independently one from another, are between 0 and 1.

According to a further particular embodiment, the catalyst has the formula Ni⁰/Co²⁺/La₂O₂CO₃.

The catalyst may be prepared by a co-precipitation method, using precursor salts of each metal contained in the catalyst to be prepared. After the synthesis, the catalytic material is optionally subjected to a pretreatment step, which includes a calcination step in air, followed by a reduction in a hydrogenating atmosphere.

Accordingly, a second aspect of the present invention relates to a process for preparing the catalyst of the invention as defined above, comprising the following steps:
i) providing an aqueous solution of a carbonate salt
ii) adding an aqueous solution of metal precursors of the first, second and third metals M1, M2 and M3;
iii) maintaining the pH between 9 and 11, by adding a solution of NaOH;
iv) filtering and washing the obtained precipitate with deionized water;
v) drying the washed precipitate;
vi) optionally, milling the washed precipitate;
vii) optionally, calcining in the presence of air at a temperature between 250 and 500 °C;
viii) submitting to a reduction in the presence of hydrogen at a temperature between 250 and 500 °C.

In step i) as defined above, the carbonate salt may preferably be selected from the group consisting of alkali metal carbonates, alkaline earth metal carbonates and ammonium carbonate, preferably from sodium carbonate, potassium carbonate and ammonium carbonate.

In step ii), "metal precursor" preferably refers to the group consisting of hydrated nitrates (e.g. Ni(NO₃)₂ · 6H₂O, Co(NO₃)₂ · 6H₂O, La(NO₃)₃ · 6H₂O), carbonates (e.g. NiCOs, CoCOs, La₂(CO₃)₃) or chlorides, preferably nitrates.

According to a preferred embodiment, the process comprises calcination step vii); although the step is not essential for the catalyst to show an acceptable performance and yield, the calcination step has shown to improve the catalyst properties even further.

According to preferred embodiments, step vii) is performed at a temperature of between 250 and 450°C, or between 300 and 450°C, or between 300 and 400°C, or between 350 and 400°C.

A third aspect of the present invention relates to an alternative process for preparing the catalyst of the invention as defined above, comprising the following steps:
i') providing a mixture of metals M2 and M3 in solid form;
ii') providing an aqueous solution of a precursor of metal M1;
iii') mixing metals M2 and M3 with the aqueous solution of metal M1, so that the metal M1 is impregnated on the solid mixture of metals M2 and M3;
iv') drying the material to remove excess of water;
v') calcining in the presence of air at a temperature between 150 and 500 °C, preferably between 300 and 450 °C;
vi') submitting to a reduction in the presence of hydrogen at a temperature between 250 and 500 °C.

A further aspect of the present invention is a method for ring-opening an oxygen-containing five or six membered heterocyclic compound, to obtain a diol,
comprising the steps of:
a) feeding the oxygen-containing five or six membered heterocyclic compound to a reactor containing the catalyst of the invention, the heterocyclic compound being in its pure form or as a solution in water, ethanol, methanol, 1-propanol, 2-propanol, 2-butanol, toluene, 1,4-dioxane, n-hexane, n-heptane or cyclohexane;
b) providing the reactor with a hydrogen pressure between 10-200 bar;
c) adjusting the temperature of the reactor between 120-220°C, to obtain the diol.

By performing the above-defined steps, the diol corresponding to the oxygen-containing 5 or 6 membered heterocyclic compound fed to the process in step a) is obtained; as necessary, the obtained diol may be separated from any unreacted heterocyclic compound, catalyst, and, if present, solvent which may be present; the separation may be performed by the use of conventional process, such as e.g. distillation. Any unreacted heterocyclic compound and solvent may be recycled back to the reactor.

In the frame of the present invention, the oxygen-containing five or six membered heterocyclic compound is a substituted, saturated or unsaturated cyclic compound, which has atoms of carbon and oxygen as members of their rings. The ring is substituted by a substituent containing an oxygen atom, selected from hydroxy, hydroxymethyl, oxo and aldehyde. Preferably, the ring contains one oxygen atom. The ring may be further substituted by a methyl or hydroxymethyl group. Preferably, the heterocyclic compound has between 4 and 6 carbon atoms.

Preferably, the oxygen-containing five or six membered heterocyclic compound is selected from the group consisting of tetrahydrofurfuryl alcohol, furfural, furfuryl alcohol, tetrahydropyran-2-methanol, 2-hydroxy-tetrahydropyran, 5-methyl tetrahydrofurfuryl alcohol, 2,5-bis(hydroxymethyl)tetrahydrofuran, gamma-valerolactone, gamma-butyrolactone, preferably tetrahydrofurfuryl alcohol.

Preferably, the method is performed with the oxygen-containing five or six membered heterocyclic compound in pure form, that is, in the absence of any solvent.

As an example, if the heterocyclic compound fed into the reactor is tetrahydrofurfuryl alcohol, 1,5-pentanediol is obtained. Thus, the method of the present invention is useful to obtain 1,5-pentanediol from tetrahydrofurfuryl alcohol.

In the above method, the reactor may be selected from continuous, semi-continuous or discontinuous reactors.

According to a preferred embodiment, the raising of hydrogen pressure is preferably preceded by purging the reactor with hydrogen. The hydrogen pressure is preferably between 10 and 150 bar, between 10 and 100 bar, between 20 and 100 bar, between 20 and 70 bar, between 30 and 100 bar or between 30 and 70 bar.

According to preferred embodiments, the temperature is maintained at 120 to 200°C, 150 to 220°C or 150 to 200 °C.

As noted above, the method may be used to convert tetrahydrofurfuryl alcohol to 1,5-pentanediol, with selectivities higher than 94 % and high activities of around 0.5 t_{product} t_{cat}⁻¹ h⁻¹). The trifunctional catalyst of the present invention is preferably based on non-noble metals, which are relatively cheap (Ni: 0.021 USD g⁻¹, Co: 0.027 USD g⁻¹, La: 0.005 USD g⁻¹). Furthermore, the catalyst may operate in moderate conditions (T = 150 - 200 °C, P = 30 - 70 bar). The catalyst of the present invention falls within the framework established as suitable according to J. Lange [Jean-Paul Lange, Catalysis Science & Technology (2016), 6, 4759], wherein the necessary criteria for the applicability of a catalyst to the biorefinery industry is examined.

### BRIEF DESCRIPTION OF THE DRAWINGS

To complete the description and in order to provide a better understanding of the invention, a set of drawings is provided. Said drawings form an integral part of the description and illustrate embodiments, which should not be interpreted as restricting the scope of the invention, but just as examples of how the invention can be carried out. The drawings comprise the following figures:
**Figure 1****:** scatter graph representing the tetrahydrofurfuryl alcohol conversion without solvent over time.
   Catalyst: NiOₓ/CoOₓ/La₂O₂CO₃.
   Feed = 10.54 g tetrahydrofurfuryl alcohol, 1 g catalyst, 54 bar, 180 °C.
**Figure 2****:** bar graph representing the tetrahydrofurfuryl alcohol conversion and yield of the different obtained products, for catalysts according to the present invention having different molar ratio of Ni to Co.
   Catalyst: NiOₓ/CoOₓ/La₂O₂CO₃.
   Feed = 0.4 g tetrahydrofurfuryl alcohol, 10 mL ethanol, 0.1 g catalyst, 54 bar, 180 °C, 24 h.
**Figure 3****:** bar graph representing the tetrahydrofurfuryl alcohol conversion and yield of the different obtained products, for catalysts according to the present invention with different calcination temperatures.
   Catalyst: NiOₓ/CoOₓ/La₂O₂CO₃.
   Feed = 0.4 g tetrahydrofurfuryl alcohol, 10 mL ethanol, 0.1 g catalyst, 54 bar, 180 °C, 24 h.
**Figure 4****:** bar graph representing the tetrahydrofurfuryl alcohol conversion and yield of the different obtained products, for catalysts according to the invention submitted to different reduction temperatures.
   Catalyst: NiOₓ/CoOₓ/La₂O₂CO₃.
   Feed = 0.4 g tetrahydrofurfuryl alcohol, 10 mL ethanol, 0.1 g catalyst, 54 bar, 180 °C, 24 h.

### EXAMPLES

The following examples serve to further describe and illustrate the present invention, but in no way should they be construed as limiting the scope of the invention.

### Example 1: preparation of a catalyst according to the present invention

The synthesis of a NiOₓ/CoOₓ/La₂O₂CO₃ catalyst according to the present invention, with a molar ratio of 2/2/1, was carried out by co-precipitation method. A 2M aqueous Na₂CO₃ solution was prepared in a stirred vessel. Subsequently, a 100 mL aqueous solution containing the adjusted concentration of metal nitrates (6.2 g of nickel nitrate, 6.3 g of cobalt nitrate and 4.6 g of lanthanum nitrate) was added dropwise. Simultaneously, a 2M NaOH aqueous solution was used to adjust the pH to 10 ± 0.1. The suspension was allowed to age for 24 hours at 80°C; subsequently, the solid was filtered and washed with deionized water, until the filtrate reached a pH of 7. The resulting material was dried for 24 hours at 100°C. The material was ground and sieved to obtain a particle size of ≤ 0.2 mm. The metal oxide was obtained by calcination under an air flow of 100 mL min⁻¹ at a temperature of 450 °C for 4 hours, with a heating ramp of 4 °C min⁻¹. Finally, the metal oxide was reduced under a 100 mL min⁻¹ flow of 10% H₂/N₂, at a temperature of 450 °C for 4 hours, and with a heating ramp of 4 °C min⁻¹.

### Example 2: preparation of catalysts with different Ni:Co molar ratios

The method described in Example 1 was repeated by using different Ni:Co molar ratios, thus preparing different catalysts; in particular, the following NiOₓ/CoOₓ/La₂O₂CO₃ catalysts were prepared:

**Table 1:**

| **Molar ratio Ni:Co:La** | **Weight ratio of metal nitrate used for the precipitation (g)** |
|---|---|
| 0:4:1 | 0:12.5:4.6 |
| 1:3:1 | 3.1:9.4:4.6 |
| 2:2:1 | 6.2:6.3:4.6 |
| 3:1:1 | 9.4:3.1:4.6 |
| 4:0:1 | 12.5:0:4.6 |

As may be observed, in comparative catalyst 1, Nickel is absent, and in comparative catalyst 2, Cobalt is absent.

### Example 3: catalytic activity in the ring opening of tetrahydrofurfuryl alcohol in absence of solvent

10 mL of tetrahydrofurfuryl alcohol and 1 g of NiOₓ/CoOₓ/La₂O₂CO₃ catalyst with a molar ratio of 1/3/1 were added to a 30 mL stainless steel batch reactor, equipped with a magnetically driven stirrer. The reactor was sealed, purged three times with H₂, and then pressurized to 30 bar. Subsequently, the reactor was heated to the required temperature (around 180 °C) with a stirring speed of 500 rpm. The reactor pressure was kept constant by semicontinuous feeding of H₂. After the reaction (4 - 30 h), the reactor was cooled to 50 °C. The solid-liquid mixture was filtered with a 0.22 µm PTFE filter. Before analysis, 0.12 g of dodecane was added to the liquid mixture as an internal standard for gas chromatography analysis. Liquid products were analyzed on an Agilent 7890 gas chromatograph with a DB-1 column (60 m × 530 µm × 5 µm) and a flame ionization detector (FID).

In the following table, the conversion rate and yields of the different obtained products are indicated, at different reaction times.

**Table 2:**

| **Reaction time (h)** | **Conversion (%)** | **R_{1,5-PDO} (%**) | **R_{1-BOH} (%**) | **R**_{**1**-}**_{POH} (%**) | **Rₒₜₕₑᵣₛ (%**) |
|---|---|---|---|---|---|
| 4 | 16.5 | 16.4 | 0.0 | 0.0 | 0.1 |
| 8 | 25.5 | 24.5 | 0.4 | 0.3 | 0.2 |
| 18 | 51.0 | 48.0 | 1.8 | 1.0 | 0.2 |
| 24 | 66.0 | 62.4 | 3.2 | 0.3 | 0.1 |
| 30 | 90 | 85.5 | 3.5 | 0.4 | 0.6 |

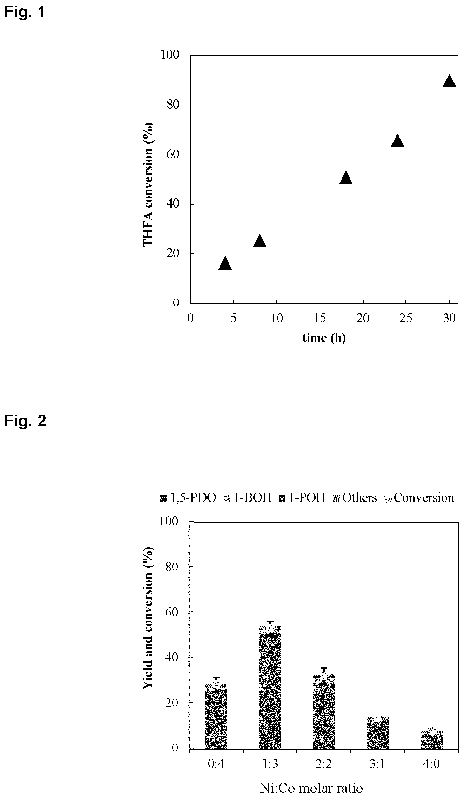

The data are graphically represented in Figure 1.

### Example 4: catalytic activity of different Ni:Co ratios

Although the method of ring opening the oxygen-containing five or six membered heterocyclic compound is preferably performed in absence of any solvent, in order to be able to compare the performance of the catalyst according to the present invention and state of the art catalysts, the following assay was performed using tetrahydrofurfuryl alcohol diluted in ethanol.

In particular, 0.4 g tetrahydrofurfuryl alcohol in 10 mL ethanol was reacted in the presence of 0.1 g of the respectively tested catalyst (Ni⁰/Co²⁺/La₂O₂CO₃ with different Ni:Co molar ratios), at a temperature of 54 bar and 180 °C, during 24 h.

Table 3 presents the conversion rates and yields of the products obtained after the ring-opening reaction for each of the tested catalysts.

**Table 3:**

| **Catalyst Ni:Co molar ratio** | **Conversion (%**) | **R_{1,5-PDO} (%)** | **R_{1-BOH} (%**) | **R**_{**1**-}**_{POH} (%**) | **Rₒₜₕₑᵣₛ** (%) |
|---|---|---|---|---|---|
| 0:4 | 28.6 | 26.2 | 0.7 | 0.0 | 1.7 |
| 1:3 | 53.2 | 51.4 | 1.0 | 0.8 | 0.8 |
| 2:2 | 32.4 | 29.2 | 2.2 | 0.9 | 1.0 |
| 3:1 | 13.8 | 12.6 | 0.0 | 0.0 | 1.3 |
| 4:0 | 7.9 | 6.4 | 0.7 | 0.0 | 0.8 |

The data are graphically represented in Figure 2.

### Example 5: catalytic activity at different calcination temperatures of the catalyst of the invention

Although the method of ring opening the oxygen-containing five or six membered heterocyclic compound is preferably performed in the absence of any solvent, in order to be able to compare the performance of the catalyst according to the present invention and state of the art catalysts, the following assay was performed using tetrahydrofurfuryl alcohol diluted in ethanol.

In particular, 0.4 g tetrahydrofurfuryl alcohol in 10 mL ethanol was reacted in the presence of 0.1 g of the catalyst (Ni⁰/Co²⁺/La₂O₂CO₃), at a temperature of 54 bar and 180 °C, during 24 h.

In Table 4, the conversion rates and yields of the different products obtained after the ring-opening reaction are gathered for each of the tested catalysts, subjected to different calcination temperatures as indicated:

**Table 4:**

| **Catalyst Calcination temperature (°C)** | **Conversion (%)** | **R_{1,5-PDO} (%)** | **R_{1-BOH} (%)** | **R**_{**1**-}**_{POH} (%)** | **Rₒₜₕₑᵣₛ (%)** |
|---|---|---|---|---|---|
| DR | 75.6 | 67.7 | 5.2 | 1.4 | 1.3 |
| 250 | 81.1 | 74.1 | 5.9 | 0.7 | 0.3 |
| 350 | 68.5 | 61.2 | 3.6 | 0.7 | 3.0 |
| 450 | 53.2 | 51.4 | 1.0 | 0.9 | 0.0 |

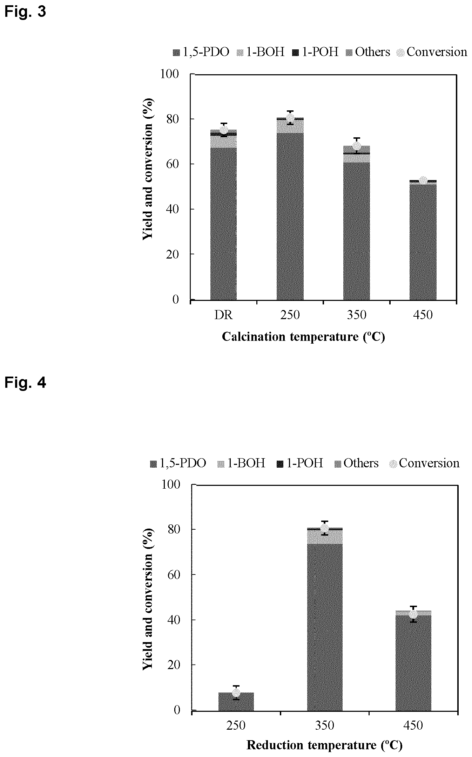

The data are graphically represented in Figure 3.

### Example 6: catalytic activity at different reduction temperatures of the catalyst of the invention

Although the method of ring opening the oxygen-containing five or six membered heterocyclic compound is preferably performed in absence of any solvent, in order to be able to compare the performance of the catalyst according to the present invention and state of the art catalysts, the following assay was performed using tetrahydrofurfuryl alcohol diluted in ethanol.

In particular, 0.4 g tetrahydrofurfuryl alcohol in 10 mL ethanol was reacted in the presence of 0.1 g of the catalyst (Ni⁰/Co²⁺/La₂O₂CO₃), at a temperature of 54 bar and 180 °C, during 24 h.

In Table 4, the conversion rate and the weight percentages of secondary products obtained after the ring-opening reaction are gathered for each of the tested catalysts, submitted to different reduction temperatures as indicated:

**Table 5:**

| **Catalyst Reduction temperature (°C)** | **Conversion (%)** | **R_{1,5-PDO} (%)** | **R_{1-BOH} (%)** | **R**_{**1**-}**_{POH} (%)** | **Rₒₜₕₑᵣₛ (%)** |
|---|---|---|---|---|---|
| 250 | 8.2 | 8.2 | 0.0 | 0.0 | 0.0 |
| 350 | 81.1 | 74.1 | 5.9 | 0.7 | 0.3 |
| 450 | 43.0 | 42.5 | 1.5 | 0.0 | 0.4 |

The data are graphically represented in Figure 4.

## Claims

1. A catalyst comprising:
1) a first metal M1, selected from Nickel, Copper, Platinum, Ruthenium, Rhodium, Iridium, Palladium and mixtures of two or three of the foregoing;
2) a second metal M2, which is Cobalt;
3) a third metal M3, selected from Lanthanum, Cerium, Yttrium, Samarium, Praseodymium, Neodymium and mixtures of two or three of the foregoing;
wherein the first and second metals M1 and M2 are present as metal oxides, elemental metals, or mixtures thereof, and the third metal M3 is present as metal oxide, metal carbonate, metal hydroxide, elemental metal, or mixtures thereof.

2. A catalyst according to claim 1, comprising from 0.5 to 40 % elemental weight of M1, from 20 to 80 % elemental weight of M2, and from 10 to 80% elemental weight of M3.

3. A catalyst according to any one of claims 1 and 2, wherein the elemental molar ratio of the first metal M1 to the second metal M2 is between 1:50 and 4:1.

4. A catalyst according to any one of claims 1 to 3, wherein the first metal M1 is nickel.

5. A catalyst according to any one of claims 1 to 4, wherein the third metal M3 is lanthanum.

6. A catalyst according to any one of claims 1 to 5, the catalyst having formula:
NiOₓ/CoO_{y}/La₂O₂CO₃
wherein x and y, independently one from another, are between 0 and 1.

7. Process for preparing the catalyst according to any one of claims 1 to 6, comprising the following steps:
i) providing an aqueous solution of a carbonate salt;
ii) adding an aqueous solution of metal precursors of the first, second and third metals M1, M2 and M3;
iii) maintaining the pH between 9 and 11, by adding a solution of NaOH;
iv) filtering and washing the obtained precipitate with deionized water;
v) drying the washed precipitate;
vi) optionally, milling the washed precipitate;
vii) optionally, calcining in the presence of air at a temperature between 250 and 500 °C;
viii) submitting to a reduction in the presence of hydrogen at a temperature between 250 and 500 °C.

8. Process according to claim 7, wherein the carbonate salt of step i) is selected from the group consisting of alkali metal carbonates, alkaline earth metal carbonates and ammonium carbonate, preferably from sodium carbonate, potassium carbonate and ammonium carbonate.

9. Process according to any one of claims 7 and 8, wherein the metal precursor of step ii) is selected from hydrated nitrates, carbonates or chlorides of the first, second and third metals M1, M2 and M3, preferably nitrates thereof.

10. Process according to any one of claims 7 to 9, wherein step vii) is present and preferably performed at a temperature between 300 and 450 °C.

11. Process for preparing the catalyst as defined in any one of claims 1 to 6, comprising the following steps:
i') providing a mixture of metals M2 and M3 in solid form;
ii') providing an aqueous solution of a precursor of metal M1;
iii') mixing metals M2 and M3 with the aqueous solution of metal M1, so that the metal M1 is impregnated on the solid mixture of metals M2 and M3;
iv') drying the material in order to remove excess of water;
v') calcining in the presence of air at a temperature between 150 and 500 °C;
vi') submitting to a reduction in the presence of hydrogen at a temperature between 250 and 500 °C, preferably between 300 and 450 °C.

12. A method for ring-opening an oxygen-containing five or six membered heterocyclic compound to obtain a diol, comprising the steps of:
a) feeding the oxygen-containing five or six membered heterocyclic compound to a reactor containing the catalyst as defined in any one of claims 1 to 6, the heterocyclic compound being in its pure form or as a solution in water, ethanol, methanol, 1-propanol, 2-propanol, 2-butanol, toluene, 1,4-dioxane, n-hexane, n-heptane or cyclohexane;
b) providing the reactor with a hydrogen pressure between 10-200 bar;
c) adjusting the temperature of the reactor between 120-220°C, thus obtaining the diol.

13. The method according to claim 12, comprising a further step of separating the oxygen-containing five or six membered heterocyclic compound from any unreacted heterocyclic compound, catalyst and solvent, if present.

14. The method according to any one of claims 12 and 13, wherein the oxygen-containing five or six membered heterocyclic compound is selected from the group consisting of tetrahydrofurfuryl alcohol, furfural, furfuryl alcohol, tetrahydropyran-2-methanol, 2-hydroxy-tetrahydropyran, 5-methyl tetrahydrofurfuryl alcohol, 2,5-bis(hydroxymethyl)tetrahydrofuran, gamma-valerolactone, gamma-butyrolactone, preferably tetrahydrofurfuryl alcohol.

15. Use of a catalyst as defined in any one of claims 1 to 6, for ring-opening of an oxygen-containing five or six membered heterocyclic compound.
